# EUROPEAN PATENT SPECIFICATION

(11) **EP 2 083 892 B1**
(45) Date of publication and mention of the grant of the patent: **25.05.2016**
(21) Application number: 07864704.7
(22) Date of filing: 21.11.2007
(51) Int. Cl.: A61M 5/315, A61C 5/06

(54) **PISTON, AND HANDHELD DISPENSER INCLUDING A PISTON**
KOLBEN UND TRAGBARER SPENDER MIT EINEM KOLBEN
PISTON, ET DISTRIBUTEUR À MAIN COMPRENANT UN PISTON

(30) Priority: 24.11.2006 US 860934 P
(43) Date of publication of application: 05.08.2009
(73) Proprietor: 3M Innovative Properties Company, St. Paul, MN 55133-3427 (US)
(72) Inventor: DUBBE, John W., 82229 Seefeld (DE); RAIA, Gioacchino, 82229 Seefeld (DE); ALBRECHT, Dirk, 82229 Seefeld (DE); LUDSTECK, Manfred, 82229 Seefeld (DE); BROYLES, Bruce R., Saint Paul, Minnesota 55133-3427 (US); PAUSER, Helmut, 82229 Seefeld (DE); GYDESEN, Henry C., Saint Paul, Minnesota 55133-3427 (US); HECHT, Reinhold, 82229 Seefeld (DE)
(74) Representative: Hohmann, Arno
(86) International application number: PCT/US2007/085331
(87) International publication number: WO 2008/064283

(56) References cited:
- EP-A- 0 879 611
- EP-A- 1 547 633
- EP-A- 1 849 490
- WO-A-97/11728
- DE-A1-102004 040 969
- GB-A- 594 003
- GB-A- 870 669
- GB-A- 1 106 825
- US-A- 5 735 437

## Description

### Technical Field

A piston, and a handheld dispenser including a piston, for use in dispensing materials such as dental adhesives or restorative products by hand.

### Background of the Invention

Handheld dispensers have been used by dental professionals for many years to dispense materials such as adhesives or restorative products into or onto a desired location. One common dispenser for this purpose is a syringe-style dispenser, which includes a cartridge having a chamber with an opening. The material is provided within the chamber, and a piston including one or more O-ring seals is provided at the rear of the chamber. To dispense the material, a user manually pushes a plunger against the piston, and the piston moves forward to expel the material from the opening.

Syringe-style dispensers work reasonably well for materials having a relatively low viscosity, but when they are used with higher-viscosity materials certain difficulties can arise. One is that the force required to advance the material can be greater than the force that the O-ring piston seal is designed to withstand, and as a result the material bypasses the O-ring toward the rear of the piston rather than being expelled through the opening at the front of the chamber. One apparent solution to this problem - making the O-ring seal larger, so that it can withstand higher pressure without allowing the material to leak - tends to make the dispenser more difficult to operate because of the corresponding increase in the force required to initiate forward movement of the piston and the material.

Another proposed improvement to syringe-style dispensers has been to substitute a rubber plug for the piston and O-ring arrangement. Rubber plugs can be advanced using a plunger in the same manner as described above, and they typically provide a continuous seal along the interface between the wall of the plug and the wall of the chamber. On the other hand, the break-away force required to initiate movement of the plug can be quite high. This is due to the relatively large surface area at which the plug contacts the chamber wall along with the material properties of such a plug, and when taken together with a highly-viscous material in the chamber, the forces required to advance the material can become quite high.

In view of these and other disadvantages associated with some common handheld dispensers of the type used to dispense dental materials in particular, it would be desirable to provide an improved dispenser and the components therefor.

A handheld dispenser for dispensing relatively low viscosity material is disclosed in US 5,735,437. The dispenser includes a generally U-shaped plunger with a pair of plunger rods that are movable in a chamber that contains the material. Each plunger rod has a groove for receiving an O-ring.

### Summary of the Invention

In a first aspect the present invention is related to a piston for use in expelling a material from a chamber. The piston comprises a piston body that has a side wall, and further a front seal. The front seal is preferably adapted for sealing a gap between the side wall of the piston body and a wall of the chamber. Preferably the front seal has a leading edge included angle, and a trailing edge included angle that is smaller than the leading edge included angle.

The front seal is defined as the seal of the piston that is adapted to be in direct contact with the material that may be filled in the chamber. Preferably the front seal comprises an annular ridge having a generally "V" shaped profile tapering off to the outer periphery of the seal.

The leading edge corresponds to the flank of the "V" shaped part of the front seal profile that is adapted to face the material in the chamber. Accordingly, the trailing edge is the edge opposite to the leading edge adapted to face away from the material in the chamber.

In a preferred embodiment of the invention the leading edge included angle is less than 90 degrees and more preferably is between about 50 degrees and about 70 degrees. In particular, the leading edge included angle is preferably between about 55 degrees and about 65 degrees and most preferably approximately 60 degrees.

In another embodiment of the invention the trailing edge included angle is between about 45 degrees and about 65 degrees and more preferably between about 50 degrees and about 60 degrees. In particular, the trailing edge included angle is approximately 55 degrees.

In a further embodiment of the invention the piston also comprises a rear seal for sealing a gap between the side wall of the piston body and a wall of the chamber. The rear seal has preferably a leading edge included angle and a trailing edge included angle, and the trailing edge included angle is between about 70 degrees and 90 degrees. In particular, the trailing edge included angle of the rear seal is preferably approximately 90 degrees.

In another embodiment of the invention the piston is made of low-density polyethylene (LDPE). Preferably, the piston is made from a LDPE grade as used for melt blown films. The piston has a preferably a Shore D hardness of between 85 and 95 (at 20°C).

In an embodiment of the invention the piston has a front seal with a convex outermost edge. The outermost edge of the front seal may also be flat. Preferably, for example for a piston body having a diameter of between 7 and 9 mm, the front seal projects form the side wall of the piston body by between 0.8 mm and 1.4 mm. and most preferably by approximately 1.1 mm.

In another embodiment of the invention the interior of the piston is hollow, and therefore adapted to receive the leading edge of a plunger. Preferably, the piston has a bore for receiving a pin of a plunger. The piston may be combined with a plunger, thus forming a unit for expelling the material from the chamber.

The piston preferably has a piston body that is substantially cylindrical. However, other suitable shapes, like a body having an elliptical or non-uniformly curved cross-section, are included. The piston may comprise a coaxial pin protruding from the piston front.

In a preferred embodiment the piston is combined with a dispenser cartridge that includes the chamber. In such combination the chamber preferably includes a material with a viscosity of between 30 Pa s and 1500 Pa s, preferably between 35 and 200 Pa s (measured at a constant shear rate of 6 s⁻¹.)

In a preferred embodiment of the invention the combination of the plunger and the dispenser cartridge comprises is a dental material, preferably a material comprising a glass ionomer cement.

A second aspect of the invention is directed to a device for expelling a material. The device comprises
(a) a cartridge having a chamber for containing the material, and an opening through which the material may be expelled; and
(b) a piston according to the invention adapted for insertion into and reciprocal movement within the chamber.

Preferably the device further comprises a plunger adapted for advancing the piston, wherein the plunger may also comprise a ratchet. The device comprises preferably an actuator system for advancing the plunger. Preferably the actuator system comprises an advancing mechanism, for example for advancing the plunger, and preferably also a stop. Furthermore, the actuator system comprises preferably a lever for manual actuation of the advancing mechanism.

In a preferred embodiment of the invention the device comprises an advancing mechanism that is adapted to alternately move a pawl in forward and return strokes for stepwise moving the plunger forward during the forward strokes. Preferably, the lever co-operates with the pawl in that it causes the pawl to move forward upon pushing the lever down. Accordingly, the pawl may be caused to move backward upon releasing the lever. Preferably, the advancing mechanism provides for (i) the pawl being in engagement with the ratchet of the plunger during the forward strokes, and for (ii) the pawl being in disengagement from the ratchet of the plunger during the return strokes. In other words, the pawl may engage with a tooth of the ratchet and therefore advance the plunger during the forward stroke while the plunger remains in its position during the return stroke. During the return stroke the pawl may slide or jump over one or more teeth of the ratchet and thereby move relative to the plunger.

In another embodiment of the invention the device comprises an actuator system that provides for (i) the stop being in engagement with the ratchet of the plunger at the end of a return stroke, and for (ii) the stop being in disengagement from the ratchet during the major part of the forward and the return strokes. In other words, the stop is preferably generally engaged when the lever is released while it becomes preferably disengaged upon pushing the lever down.

In still another embodiment of the invention the piston hinders the plunger to move back during the return strokes. This means that the plunger is preferably held in place by help of the piston so that the pawl of the actuator system can move relative to the plunger in the return stroke. Preferably the distance the pawl moves relative to the plunger is greater than the distance the plunger is moved during the forward stroke. Therefore the forward stroke of the pawl may include an idle stroke which provides for allowing the pawl to engage with a tooth of the ratchet.

In a further embodiment of the invention a device for expelling a material is provided. The device preferably comprises:
(a) a cartridge having a chamber for containing the material, and an opening through which the material may be expelled; and
(b) a piston adapted for insertion into and reciprocal movement within the chamber, the piston including
   (i) a body having at least one side wall, and
   (ii) a circumferential front seal for sealing a gap between the side wall and a wall of the chamber, the front seal having a leading edge for contact with the material.

Preferably the force required to move the piston backward is greater than the force required to move the piston forward.

A third aspect of the invention is directed to a method of providing a device for expelling a material, comprising:
(i) providing a device having a chamber with an opening, and a piston according to the invention;
(ii) positioning the piston in the chamber adjacent the opening; and
(iii) injecting the material through the opening and into the chamber, thereby pushing the piston backward.

In another embodiment of the invention a method of filling a cartridge is provided comprising the steps of:
(i) placing a hollow piston according to the invention to the front most position in a chamber of a cartridge;
(ii) injecting a material through the opening of the chamber and into the chamber, thereby pushing the piston backward; and
(iii) inserting the front part of a plunger into the hollow space of the piston.

Preferably, the piston is expanded by the front part of the piston, for example so that the contact pressure of the piston seals against the chamber wall is increased.

### Brief Description of the Drawings

The present invention will be described with reference to the Figures, in which:
Figure 1 is a schematic cross-sectional view of a piston disposed in a chamber of a cartridge according to an embodiment of the invention;
Figure 2 is a cross-sectional side view of a hollow piston according to an embodiment of the invention;
Figures 2a, 2b are schematic partial side views of a front seal according to an embodiment of the invention;
Figure 3 is a schematic illustration showing the configuration of the including angles of a leading and trailing edge of the piston according to an embodiment of the invention;
Figure 4 is a side view of a piston having additional intermediate seals according to an embodiment of the invention;
Figure 5 is a cross-sectional view of a cartridge including a chamber with a piston combined with a plunger according to an embodiment of the invention;
Figure 6 is a schematic cross-sectional view of a piston combined with a plunger according to an embodiment of the invention;
Figure 7 is a perspective view of a dispenser according to an embodiment of the invention;
Figure 8 is a perspective exploded view of a dispenser according to an embodiment of the invention;
Figure 9 is a perspective view of a detail of a dispenser according to an embodiment of the invention;
Figure 10a, 10b are schematic cross-sectional views of a piston having a pin being disposed in a chamber of a cartridge in two positions according to an embodiment of the invention;
Figure 11 is a cross-sectional view of an exemplary piston according to an embodiment of the invention; and
Figure 12 is a cross-sectional view of a comparative piston in relation to the exemplary piston of Figure 11.

### Detailed Description of the Invention

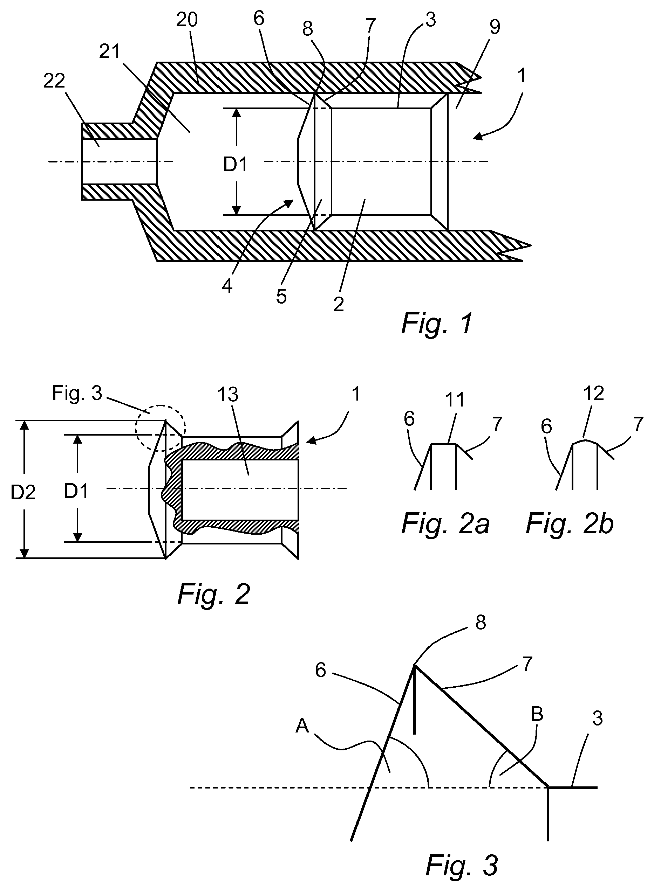

Figure 1 illustrates a section of a cartridge 20 with a chamber 21 of a dispenser and one embodiment of a piston 1 according to the present invention. The chamber is normally cylindrical, but can in principle be of any desired shape. At the front end of the chamber, cartridge 20 includes an opening 22, through which material that may be contained in chamber 21 can be expelled. The chamber can include a flat front end, to minimize the residual material that remains when the piston reaches the end of its forward travel, or it can be conical or frusto-conical, possibly corresponding to the shape of the front end of the piston. The opening may extend through a nozzle, and the nozzle may be closed with a removable cap (not shown).

The piston 1 is designed to fit within the chamber 21 and includes a piston body 2, which is typically cylindrical (and thus includes a single side wall 3) when used with a dispenser having a cylindrical chamber. The piston body 2 may be hollow (shown in Fig. 2), for example it may have a bore 13, to facilitate the insertion and retention of a plunger used to advance the piston 1, or it may be solid. The piston shown in Figure 1 also includes a beveled front surface 4, which when shaped to match the interior of the front of the chamber can reduce the amount of material left in the chamber when the piston 1 has been advanced as far as possible. As can be seen the diameter D1 of the piston body 2 is selected so that it is less than the interior diameter of the chamber 21 within which it is contained. The gap, or clearance, between the body 2 and the wall(s) of the chamber 21 is bridged by front seal 5.

In the embodiment illustrated in Figure 1, the front seal 5 has a leading edge 6, and a trailing edge 7, which meet at ridge 8. This provides for better sealing on rougher surfaces or with non-uniform mating surface geometry, such as varying internal wall diameter or roundness. Due to the pistons ability to seal uneven and non-uniform surfaces, the molding specifications can be more lenient, resulting in reduced costs.

Although the leading and trailing edges 6, 7 are shown as meeting along ridge 8, in fact the area where the two edges meet may be a flat surface, or a curved surface, as described in greater detail below. The leading edge 6 is adapted to be in contact with the material that may be held within the chamber 21, and the ridge 8 is adapted to contact the side wall(s) of the chamber. The outer diameter D2 of the ridge 8 of the front seal 5 (indicated in Fig. 2) is typically selected so that it is greater than the interior diameter of the chamber within which it is contained. Thus, the piston 1 seals with the wall(s) of the chamber 21. The piston is adapted for reciprocal (forward and backward) movement in response to the application of a sufficient force in one direction or the other.

One feature of the present invention relates to the design of the front seal and is shown in greater detail in Fig. 3. Leading edge 6 has a leading edge included angle A, which is measured relative to the side wall 3 of the piston 1, and trailing edge 7 has a trailing edge included angle B, which is also measured relative to the side wall 3 of the piston. In contrast to conventional devices in which a symmetrical front seal might have a leading edge included angle that is equal to a trailing edge included angle, the trailing edge included angle B of the front seal 5 of the present invention is smaller than the leading edge included angle A. In other words, the slope of the leading edge relative to the side wall of the piston is steeper than the slope of the trailing edge.

The leading edge included angle A may be selected as desired, and is preferably from about 50 degrees to about 70 degrees, more preferably from about 55 degrees to about 65 degrees, and most preferably about 60 degrees. Trailing edge included angle B may also be selected as desired, and is preferably from about 45 degrees to about 65 degrees, more preferably from about 50 degrees to about 60 degrees, and most preferably about 55 degrees. The ranges given above, however, shall exclude a sub-range in which the leading edge included angle A is equal or smaller than the trailing edge included angle B. The leading edge and the trailing edge may be said to exhibit a general "V" shape when viewed in cross-section, although the legs of the "V" are not symmetrical.

In the embodiment shown in Figures 1 to 3, the front seal includes a ridge 8 that contacts the wall of the chamber. It is possible that when the piston is pushed forward, the leading edge of the front seal may flex backward, so that the leading edge contacts the wall of the chamber to a greater degree than the ridge. Similarly, when the piston is pushed backward, as during a filling operation, the front seal may flex so that the trailing edge contacts the wall of the chamber. Either of these is operationally normal.

The leading edge 6 and the trailing edge 7 may adjoin each other at a surface, as shown in Figure 2a (a flat surface 11) or in Figure 2b (a curved surface 12). These surfaces and other surface modifications can either be molded into the front seal, or the front seal can be shaped as a "V" shape as described above, and then the vertex of the "V" can be milled off or otherwise manufactured. The embodiments schematically shown in Figures 2a and 2b include the leading edge 6 and the trailing edge 7 that comprise the major portion of the V-shaped front seal described above, and thus fall within the definition of a generally V-shaped front seal as that term is used herein.

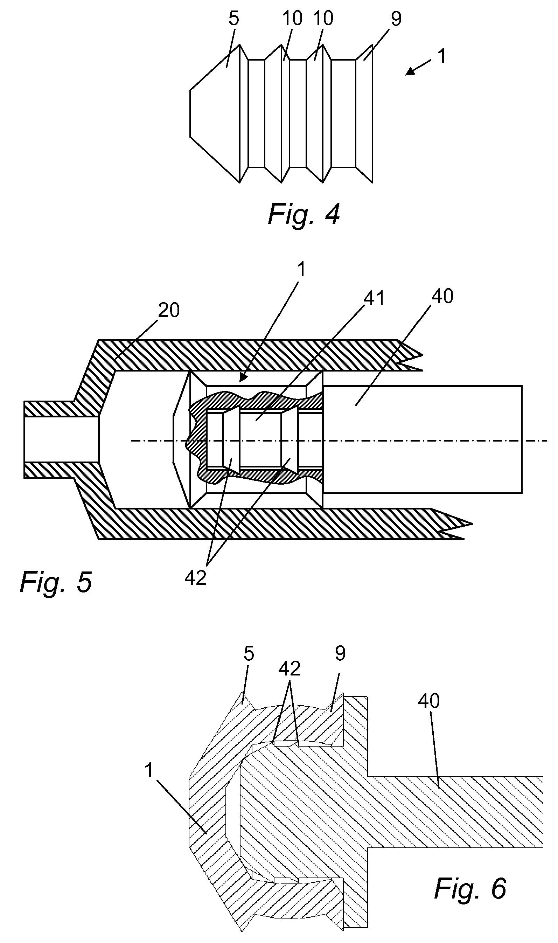

The piston of the present invention may include additional structural variations. For example, the piston 1 may include a rear seal 9, and one or more intermediate seals 10, as shown in Figure 4, to position the piston within the chamber. Those seals may be designed in the same manner as the front seal 5, or they may be designed in the same manner as conventional seals, using O-rings, for example.

The piston of the present invention may be made of any suitable material or blend of materials, including low density polyethylene (LDPE), high density polyethylene (HDPE), polypropylene (PP) or other materials having similar properties. Preferably, the piston is made of a LDPE for melt blown film, having a melt flow index of between 7 to 9, preferably 8 g/10 min (190 °C/2.16 kg, according ASTM Standard D 1238) and a density of between 0.91 to 0.94, preferably 0.922 g/cm³ (at 23 °C, according to ASTM D 1505). The dimensions of the piston are normally determined based on the amount of material to be dispensed by the dispenser, so that larger devices have larger chambers and correspondingly larger pistons. Preferably the diameter D2 of the piston 1 is between 7 mm and 9 mm and preferably 8.1 mm and the inner diameter of the chamber 21 is preferably 7.9 ± 0.05 mm.

It has been found that the combination of the piston material, the dimensions of the piston and the seal(s), as well as, the geometric configuration of the front seal provides certain effects, when extruding medium and higher viscosity materials.

For example, the piston preferably provides for backward movement at a higher force than required for forward movement. More preferably the piston is adapted to provide a certain minimum force for backward movement. This may be particularly of advantage in case the piston is used with a dispenser for incrementally or discontinuously dispensing material, for example a dispenser in which a plunger is advanced stepwise in response of actuating a lever. Usually in such a system a plunger is stepwise moved forward to advance a piston within a chamber. Such types of dispensers of prior art normally require a return lock to restrict the movement of the plunger to the forward direction because otherwise the plunger may travel back between the steps of movement. This may happen, for example because there might be a remaining pressure in the chamber that pushes the piston and plunger back to compensate. For example, pressure may be build up due to the elasticity of the cartridge, compressibility of the piston or even because of air contained in the material to be dispensed. However, the piston of the invention allows, under circumstances as described in more detail below, for elimination of the return lock because the piston is adapted to retain itself to a certain degree. When used with a dispenser of this kind the piston would preferably be connected with the plunger so that the plunger when released is held in place by the piston and therefore the return lock may be eliminated.

Usually the moving force comprises a peak force or break away force which is required to loosen the seal from its rest position at the chamber wall. Further, there is a sliding force. Normally the break away force is higher than the sliding force. For example, an O-ring usually has a relatively high break away force while the sliding force is relatively low. If an O-Ring is used in a dispenser for extruding high viscous material the extrusion force and the break away force may add up to a total force that is unacceptable. On the other hand there is a limit for the extrusion of low viscous material by use of an O-ring seal because the piston may move with a stick slip effect caused by a too low total of the sliding and extrusion force. This may cause uncontrolled dispensation or even squirting of the material. In contrast, the piston of the invention provides for a relatively low break away force which makes it useable for very high viscous materials. Further the piston of the invention provides for a sufficiently high sliding force that allows smooth movement of the piston even when used with low viscous material. Therefore the piston of the invention generally allows for dispensing of rather high viscous materials as well as for dispensing of rather low viscous materials which makes is more universally usable relative to a piston of the prior art.

As shown in Figure 5 the piston 1 may be pushed forward by a plunger 40 that is pressed against the back surface of the piston. As shown, the plunger may have a pin 41 which is inserted into the bore 13 of the piston 1. The pin may have a retainer 42 (for example one or more barbs or ridges) to hold the piston 1 on the pin 41 of the plunger 40, for example to prevent the piston from detaching when the plunger 40 is pulled backward. Preferably the plunger including the pin is made of a relatively rigid plastic material, for example acrylonitrile-butadiene-styrene (ABS), while the piston is preferably made of a softer material, like LDPE. Thus, the pin reinforces the piston in that it forms a relatively rigid core of the otherwise relatively soft piston. Therefore the overall dimensional stability of the piston is increased relative to a piston without a rigid core while particular parts, for example the seal(s), remain relatively soft. This provides the advantage that first, the relatively soft seal(s) are pressed to the wall(s) of the chamber within which the piston is contained at relatively high forces which facilitates good sealing. Second, the compressibility of the piston is lowered which generally facilitates better control of expelling precise amounts of material. This is because the length-compression of the piston is lowered relative to a piston that is not reinforced and therefore the movement of the plunger is transmitted more directly (meaning less cushioned by the piston) to the material to be dispensed.

A third advantage is given by the fact, that the piston can be used independently of the plunger during the manufacturing filling process. In a method of filling the dispenser through the outlet of the chamber, as it is described in more detail below, the piston is disposed adjacent the front end of the chamber and pushed back by the material filled into the chamber. Because the piston in that case is not reinforced the pressing force of the seal(s) to the chamber wall(s) is reduced relative the piston when assembled with the plunger. This also reduces the friction of the piston when it is pushed back and therefore allows filling of the dispenser at relatively low forces. On the other hand, as soon as the dispenser has been filled the sealing force may be increased, for example to provide a good shelf life of the material stored in the chamber, by assembly of the plunger with the piston. Further, after assembly of the plunger the piston may also have the self-retaining properties as mentioned above.

In another embodiment, as shown in Figure 6, the retainers 42 bulge out the side walls of the piston 1 which provides the effect that the seals 5, 9 are deflected away from one another. This configuration provides for a generally constant outer diameter of the seals 5, 9 independent from the use with and without the plunger 40. As an advantage the piston seals are compressed by the chamber wall(s) at a substantially equal level in both cases, when the piston is used alone and when it is used in combination with the plunger. This allows for good sealing during filling and on the other hand helps providing for relatively low forces to move the piston when used with the plunger.

The piston may be part of a larger hand-held device, so that the same plunger is used with successive containers each having its own piston, or an individual plunger may be provided and subsequently discarded with each dispenser. The plunger should be rigid enough to withstand the forces associated with the operation of the dispenser, and the plunger may be stabilized by one or more ribs or other support members.

The dispenser of the present invention may be used to dispense many different kinds of materials, including ones of low, medium and high viscosity. Examples of those materials include dental filling materials such as, for example, the glass ionomer filler material Ketac^{™} Molar, or the light-cured glass ionomer filler material Photac^{™} Fil Quick, or the silver-reinforced glass ionomer filler material Ketac^{™} Silver Molar. Further those materials may include dental luting cements such as, for example, the self-adhesive universal composite luting cement RelyX^{™} Unicem or the adhesive composite luting cement Compolute^{™} or the glass ionomer luting cement Ketac^{™} Cem. The dental materials mentioned are, for example, available from the 3M ESPE Company of Germany.

The piston and the dispenser of the present invention find particularly advantageous use in the dispensation of medium to high viscosity materials such as 3M^{™} ESPE^{™} RelyX^{™} Unicem Clicker, and in general for materials having a viscosity of between 30 to 1500 Pa s (measured at a constant shear rate of 5 s⁻¹), and in particular for materials having a viscosity of between 35 and 200 Pa s (measured at a constant shear rate of 5 s⁻¹).

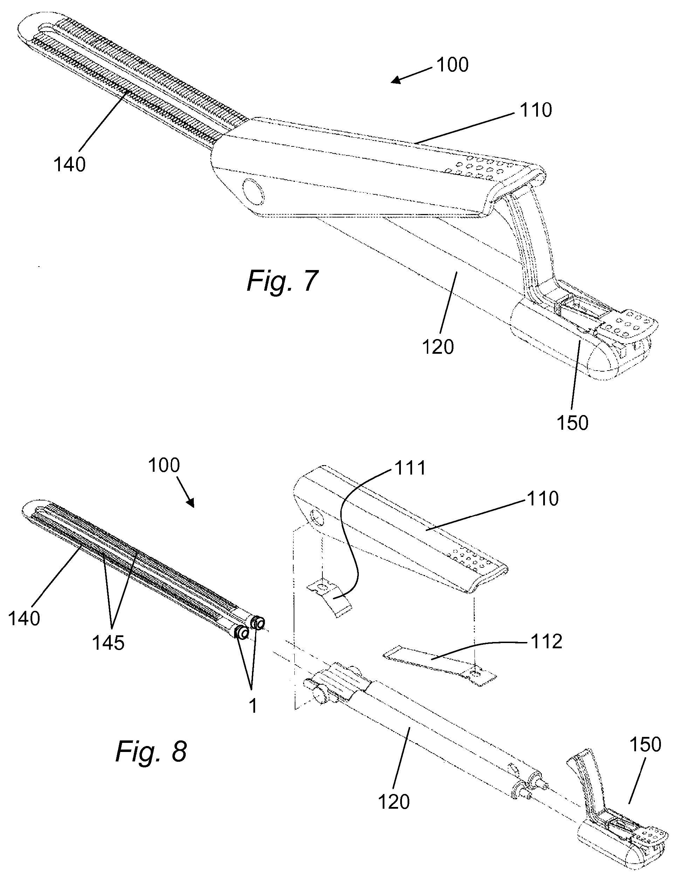

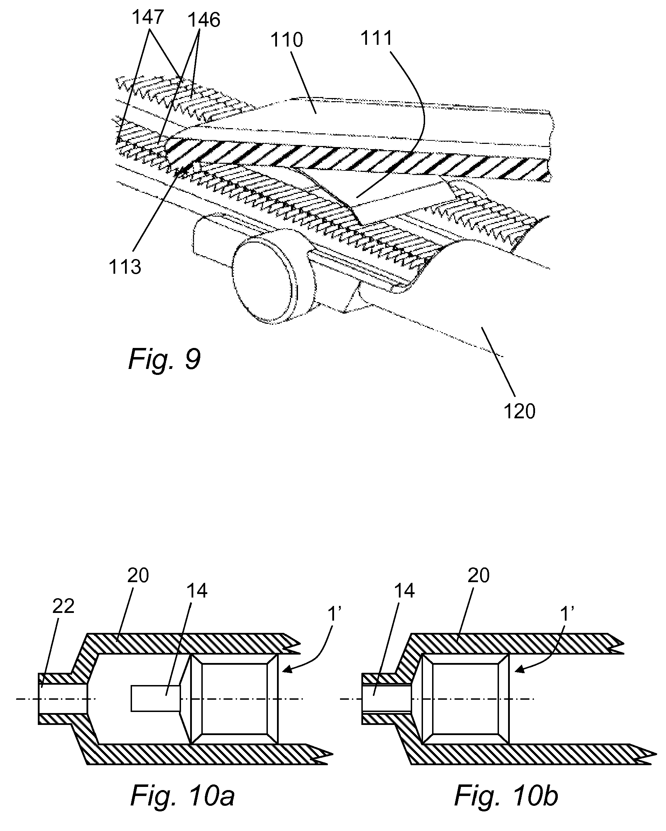

A dispenser 100 as it may be used with the piston of the invention is shown in Figure 7. The dispenser 100 comprises a cartridge 120 with two chambers in which plunger rods 145 of a plunger 140 are disposed. Further a cap 150 is shown which closes off the outlets of the cartridge 120. The plunger 140 can be moved in a stepwise manner in that the lever 110 is alternately pushed and released. The lever 110 forms a part of an actuator system which allows controlled movement of the plunger and thereby it also allows controlled dispensation of the material that may be contained in the dispenser 100. The actuator system is shown in more detail in Figures 8 and 9, wherein Figure 8 is an exploded view of the dispenser 100. Figure 8 additionally shows a pawl 111 and a return spring 112. Further, as can be seen the plunger 140 carries pistons 1 according to the invention. The plunger 140 is generally U-shaped, and thus the plunger rods 145 are coupled with each other. This helps to provide a generally simultaneous advancement of the plunger rods 145 and the pistons 1 and therefore dispensation of the material at a precise ratio. The plunger 140 has a ratchet which the pawl 111 is adapted to co-operate with. The ratchet has a relatively close pitch in particular to allow precise dispensation of low amounts of material upon a push of the lever, however, the ratchet may have any suitable pitch for dispensing larger amounts of paste. The return spring 112 provides for resetting the lever it an initial position when it is released.

Figure 9 shows the actuator system in greater detail. As can be seen the plunger has an inner ratchet 146 and a further outer ratchet 147 arranged side by side on the plunger rods 145. The teeth of the ratchets 146, 147 have a general saw tooth configuration, meaning that the teeth of the ratchet are asymmetrical with a steeper flank and a flatter flank. In other words the teeth of the ratchet are inclined. In particular the teeth of the inner ratchet 146 are inclined generally rearwardly while the teeth of the outer ratchet 147 are inclined generally forwardly. When the lever 110 is pushed the pawl engages with a tooth of the inner ratchet 146 and pushes the plunger 140 forward while when the lever is released the pawl moves back in a disengaged relationship to the plunger, normally by backwardly sliding over the teeth of the ratchet. Thus, alternately pushing and releasing of the lever causes the pawl to move in a forward and return stroke respectively and thereby moves the plunger forward in a stepwise manner. The configuration of the piston according to the invention as mentioned above preferably provides a self retaining effect in particular with regard to a backward movement. Therefore the plunger may be held in place by the piston during the return stroke of the pawl. For that reason a return lock as present at many dispensers of the prior art can preferably be saved.

The dispenser as shown in Figures 7 to 9 further comprises a stop formed by a detent 113 at the lever 110 which co-operates with the outer ratchet 147. As shown, the detent 113 engages with a tooth of the outer ratchet 147 and therefore prevents the plunger from moving forward. This is of advantage, for example, because the lock helps to prevent the plunger to be moved forward during handling or transportation of the dispenser, and therefore it helps minimizing the risk of unintentional dispensation of material from the dispenser. It can be seen that the stop is only active when the lever is substantially in its initial position, meaning in the position in which it is reset by the return spring 112 (Fig. 8). Therefore the lock does not hinder the plunger in its movement when the lever is displaced out of its substantial initial position. Further, this allows an easy design of the dispenser because the stop can be integrated at the lever as shown.

The dispenser of the present invention may be filled from the front, from the back, or even from a separate port in a side wall. Preferably the chamber of the dispenser is filled from the front side, meaning through the outlet. This can be done by placing the piston into the chamber in a front most position, meaning adjacent the outlet, and injecting paste through the outlets into the chamber. Thereby the piston is moved backward. Preferably the piston is used separated from the plunger so that it is not reinforced and therefore relatively soft which allows for backward movement at relatively low forces even though the piston may be adapted to retain itself with regard to backward movement when assembled with the plunger.

This method usually allows filling substantially without trapping air in the chamber because the piston initially displaces all air from the chamber so that it can be filled with the material substantially completely. A further embodiment as shown in Figure 10 illustrates a dispenser having a piston with a pin 14 which additionally helps to prevent air from being included in the chamber during filling because the pin displaces the air in the outlet. The cross-section of the pin 14 is preferably smaller than the cross-section of the outlet, as shown, so that the pin 14 leaves a clearance which allows material to pass. This is of advantage during filling because otherwise the pin could plug the outlet so that the piston would be moved backward before material has reached the chamber, thus causing a vacuum in the chamber and eventually air to penetrate into the chamber. On the other hand such clearance between the pin and the interior of the outlet also allows for emptying of the chamber during dispensation of the material at relatively uniform forces. In case the pin would not leave enough clearance the extrusion forces would increase as soon as the pin approaches the outlet. Or the pin could even plug the outlet so that a part of the material could not be extruded from the chamber.

In another method the chamber of the dispenser is filled from the back. This is of advantage because it allows filling of the chamber through its relatively large rear opening, for example by use of a filling needle having a relatively large inner cross-section. Therefore filling can be done rather quickly at a relatively high flow rate even for highly viscous materials.

In still another method of filling the dispenser comprises an additional inlet for filling, for example adjacent the front end of the chamber. This may combine the advantages of the front and back filling method because it allows filling under minimized air inclusion and on the other hand filling through a relatively large inlet opening. The inlet opening may be plugged or capped after filling or otherwise closed, for example by sealing.

The present invention provides the advantage of filling a dispensing device from the front at relatively low forces. Further it provides a relatively high sealing efficiency during storage of the device. It is an advantage that the piston of the invention provides for relatively low break-away forces while it provides sufficient high sliding forces. Further it is of advantage that the break-away and sliding forces for moving the piston of the invention may lie between the break-away force and the sliding force of a piston of the prior art. As another advantage the invention allows certain dispensing devices to be manufactured without a return lock. Still another advantage is provided because the invention allows a dispensing device without a return lock to be operated with materials of a relatively large viscosity range and particularly with relatively high viscosities for handheld devices. It is also an advantage that the chamber can be filled under minimized inclusion of air, and further that the chamber can be generally completely emptied at a substantially uniform force level and dispensing rate.

### Comparative Example:

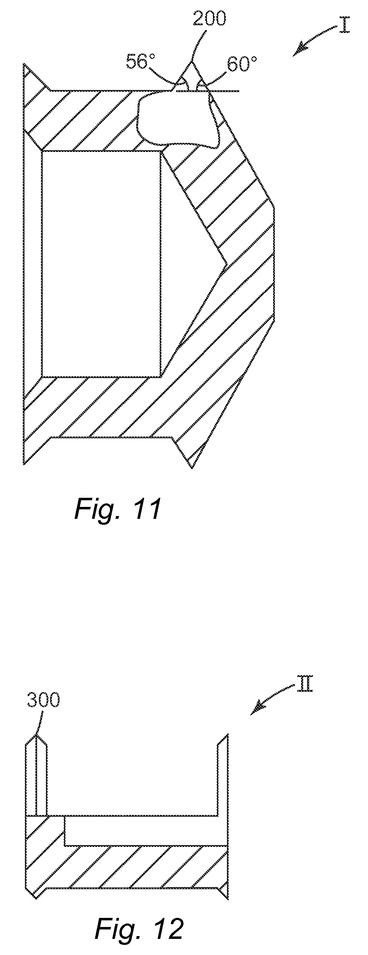

The forces occurring during a movement of two differently configured pistons in a barrel have been evaluated. Samples of a piston type I were made according to the embodiment of the invention shown in Fig. 11, and samples of a piston type II were made according to the embodiment shown in Fig. 12. The piston types I and II each had a front seal 200, 300. The front seals 200 of piston type I were made with a leading edge included angle of 60° and a trailing edge included angle of 56°. In contrast, the seals 300 of piston type II were made with a leading edge included angle of 45° and a trailing edge included angle of 45°. The samples piston types I and II were injection molded from the same plastic available from Polimeri Europa SpA, Italy, under the designation Riblene FP 20.

A barrel was made from the polypropylene plastic available from Maine Plastics Inc, Zion, Illinois under the designation LNP MF-1002 HS, with 10% glass fibers. The barrel was injection molded using this plastic material and the addition of 0.5% Kemamide® E Ultra of Chemtura Corp., Middlebury, Connecticut. The inner diameter of the barrel was dimensioned to co-operate with the pistons I and II. In particular, the inner diameter of the barrel was 7.90 mm, and therefore formed a press fit with the seals of the sample pistons, the diameter of which is indicated in Tables 1 and 2.

Five sample pistons of piston type I and five sample pistons of piston type II were provided. The seal diameters and the leading and trailing edge included angles of the piston samples were measured using a Mitutoyo profile projector.

### Test cycle:

One of the sample pistons was inserted in the barrel until it was flush with one end of the barrel, and then advanced 7 mm to an initial position in the barrel. From there the sample piston was advanced further into the barrel at a rate of 100 mm/min for a distance of 55 mm to an end position. For advancing the sample piston from the initial to the end position a Zwick Z010 Universal test machine, model number 144660 was used, and the forces applied to the sample for advancement were recorded. The advancement of the sample piston from the initial position to the end position using the Universal test machine was repeated another two times, and the sample piston was retracted to the initial position manually between tests. Therefore three measurements of the advancing force of the sample piston were obtained for each test cycle. The sample piston then was removed.

The test cycle was applied on each test sample, first oriented in the barrel for forward advancement, and subsequently repeated with the same sample oriented for backward advancement. Therefore, each of the sample pistons according to piston type I and II was advanced in the barrel three times with the front seal leading, and another three times with the front seal trailing. The corresponding forces were recorded.

The three measurements of each cycle were arithmetically averaged and recorded as "averaged forces".

All test cycles were conducted without additional lubrication of the sample pistons, and with the same barrel.

From the recorded averaged forces the following forces were determined:
1.) The break away force (the force required to cause a sample piston to move).
2.) The maximum force (the maximum force applied during the advancement of a sample piston from the initial position to the end position).
3.) The average force (the arithmetic average of the force applied during the advancement of a sample piston from the initial position to the end position).

**Table 1: Measurements for forward advancement of sample pistons according to piston type I:**

| **No.** | **Seal diameters [mm]** | | **Maximum force [N]** | **Break away force [N]** | **Average force [N]** |
|---|---|---|---|---|---|
| | **rear** | **front** | | | |
| **1** | 8,098 | 8,086 | 7,94 | 5,16 | 6,3 |
| **2** | 8,082 | 8,084 | 7,36 | 5,51 | 6,15 |
| **3** | 8,097 | 8,089 | 7,26 | 5,89 | 5,98 |
| **4** | 8,067 | 8,091 | 6,96 | 5,6 | 5,71 |
| **5** | 8,084 | 8,088 | 5,95 | 5,2 | 5 |
| **AV** | **8,086** | **8,088** | **7,09** | **5,47** | **5,83** |
| **S** | **0,013** | **0,003** | **0,73** | **0,30** | **0,51** |

**Table 2: Measurements for forward advancement of sample pistons according to piston type II_{:}**

| **No.** | **Seal diameters [mm]** | | **Maximum force [N]** | **Break away force [N]** | **Average force [N]** |
|---|---|---|---|---|---|
| | **rear** | **front** | | | |
| **1** | 8,006 | 8,198 | 10,41 | 8,68 | 9,17 |
| **2** | 7,985 | 8,165 | 9,53 | 8,9 | 8,63 |
| **3** | 8,005 | 8,173 | 9,94 | 9,15 | 8,74 |
| **4** | 8,001 | 8,123 | 9,35 | 9,17 | 8,32 |
| **5** | 8,001 | 8,175 | 10,16 | 8,75 | 8,7 |
| **AV** | **8,000** | **8,167** | **9,88** | **8,93** | **8,71** |
| **S** | **0,008** | **0,027** | **0,44** | **0,22** | **0,30** |

**Table 3: Angular measurements:**

| **Seal angles** | | | |
|---|---|---|---|
| | **Leading edge** | **Trailing edge** | **rear seal** |
| **Piston I** | 60,2° | 56,3° | 45° |
| **Piston II** | 48,3° | 42,7° | 45° |

The total averages "AV" and standard deviations "s" are calculated from the data rows 1 to 5 in the corresponding Tables 1 and 2. The following diagrams show the total averages of the break away forces, maximum forces and the average forces, and the standard deviations.

Diagram 1 shows the total average forces of the piston samples according to piston type I:

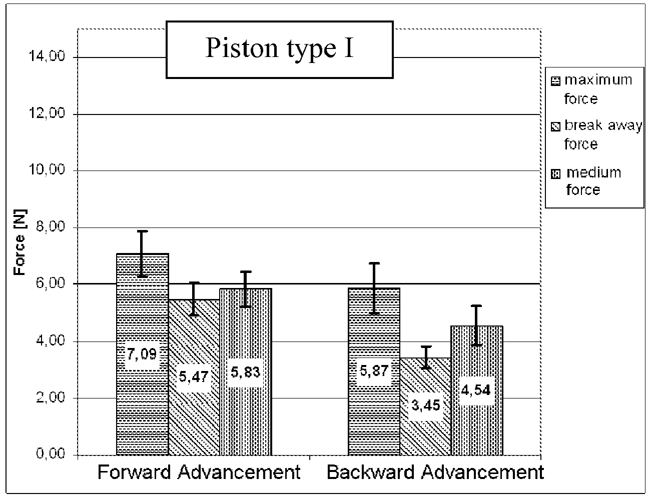

Diagram 2 shows the total average forces of the piston samples according to piston type II:

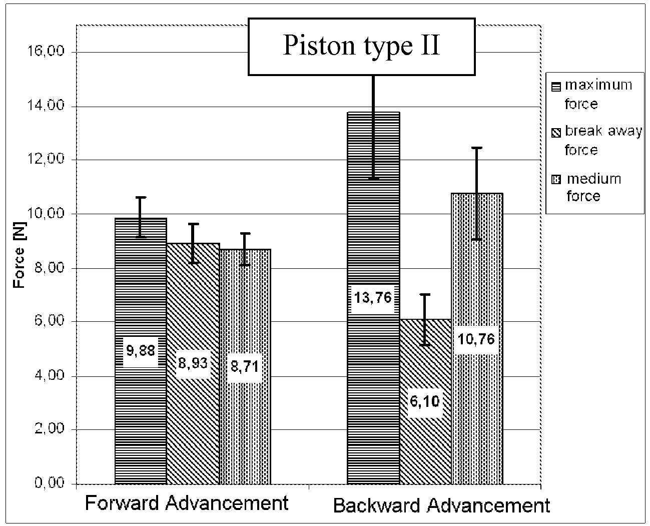

Diagram 3 shows a comparison of the forces of the piston samples between piston types I and II for forward advancement:

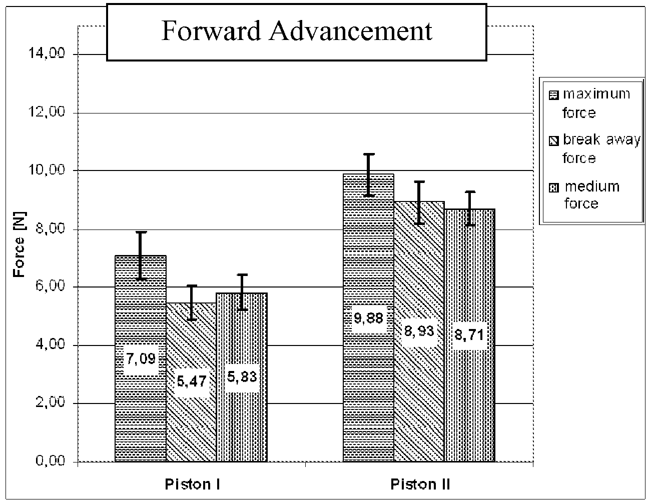

Diagram 4 shows a comparison of the forces of the piston samples between piston types I and II for backward advancement:

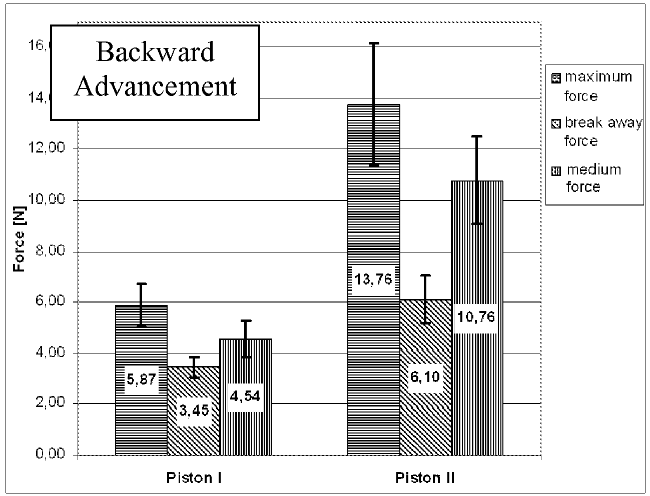

The present invention has been described with reference to several embodiments, but the invention shall not be limited by those examples, but only by the following claims and the equivalents thereof.

## Claims

1. A piston (1) for use in expelling a material from a chamber (21), comprising:
(a) a piston body (2) having a side wall (3); and
(b) a front seal (5) for sealing a gap between the side wall (3) of the piston body (2) and a wall of the chamber (21), wherein the front seal (5) comprises an annular ridge (8) having a generally "V" shaped profile tapering off to the outer periphery of the seal (5), and has a leading edge included angle (A), and a trailing edge included angle (B) that is smaller than the leading edge included angle (A), the leading and trailing edge included angles (A, B) being measured relative to the side wall (3) of the piston, wherein the leading edge (6) corresponds to the flank of the "V" shaped part of the front seal profile that is adapted to face the material in the chamber (21), and the trailing edge (7) is the edge opposite to the leading edge (6) adapted to face away from the material in the chamber (21), and wherein the leading edge included angle (A) is approximately 60 degrees, and wherein the trailing edge included angle (B) is approximately 55 degrees.

2. The piston of any of the preceding claims, wherein the piston (1) further comprises a rear seal (9) for sealing a gap between the side wall (3) of the piston body (2) and a wall of the chamber (21), wherein the rear seal (9) has a leading edge included angle and a trailing edge included angle, and the trailing edge included angle is between about 70 degrees and 90 degrees.

3. The piston of claim 2, wherein the trailing edge included angle of the rear seal (9) is approximately 90 degrees.

4. The piston of any of the preceding claims, wherein the piston (1) is made of low-density polyethylene (LDPE) and has a Shore D hardness of between 85 and 95.

5. The piston of any of the preceding claims, wherein the front seal (5) projects form the side wall (3) of the piston body (2) by between 0.8 mm and 1.4 mm.

6. The piston of any of the preceding claims, wherein the piston (1') comprises a coaxial pin (14) protruding from the piston front.

7. The piston of any of the preceding claims, in combination with a dispenser cartridge (20) that includes the chamber (21).

8. The combination of claim 7, wherein the chamber (21) includes a material with a viscosity of between 30 and 1500 Pa s.

9. The combination of claim 8, wherein the material is a dental material.

10. A device for expelling a material, comprising:
(a) a cartridge (20) having a chamber (21) for containing the material, and an opening (22) through which the material may be expelled; and
(b) a piston (1) according any of the claims 1 to 6 adapted for insertion into and reciprocal movement within the chamber (21).

11. The device of claim 10, further comprising a plunger (14) adapted for advancing the piston (1), and wherein the plunger (140) comprises a ratchet, and the device comprising an actuator system for advancing the plunger (140), and wherein the actuator system comprises an advancing mechanism and a stop, and wherein the actuator system further comprises a lever (110) for manual actuation of the advancing mechanism, and wherein the advancing mechanism is adapted to alternately move a pawl (111) in forward and return strokes for stepwise moving the plunger (140) forward during the forward strokes.

12. The device of claim 11, wherein the advancing mechanism provides for
(i) the pawl (111) being in engagement with the ratchet of the plunger (140) during the forward strokes, and for
(ii) the pawl (111) being in disengagement from the ratchet of the plunger (140) during the return strokes.

13. The device of claims 11 and 12, wherein the actuator system provides for
(i) the stop being in engagement with the ratchet of the plunger at the end of a return stroke, and for
(ii) the stop being in disengagement from the ratchet during the major part of the forward and the return strokes.

## Patentansprüche

1. Kolben (1) zur Verwendung beim Herauspressen eines Materials aus einer Kammer (21), umfassend:
(a) einen Kolbenkörper (2) mit einer Seitenwand (3); und
(b) eine vordere Dichtung (5) zum Abdichten eines Zwischenraums zwischen der Seitenwand (3) des Kolbenkörpers (2) und einer Wand der Kammer (21), wobei die vordere Dichtung (5) einen ringförmigen Grat (8) umfasst, der ein generell "V"-förmiges Profil hat, das sich zur äußeren Peripherie der Dichtung (5) hin verjüngt, und wobei sie einen an der Vorderkante eingeschlossenen Winkel (A) hat sowie einen an der Hinterkante eingeschlossenen Winkel (B), der kleiner ist als der an der Vorderkante eingeschlossene Winkel (A), wobei die an der Vorderkante und an der Hinterkante eingeschlossenen Winkel (A, B) relativ zur Seitenwand (3) des Kolbens gemessen werden, wobei die Vorderkante (6) der Flanke des "V"-förmigen Teils des Profils der vorderen Dichtung entspricht, das dazu eingerichtet ist, dem Material in der Kammer (21) zugewandt zu sein, und wobei die Hinterkante (7) die der Vorderkante (6) gegenüberliegende Kante ist, die dazu eingerichtet ist, vom Material in der Kammer (21) abgewandt zu sein, und wobei der an der Vorderkante eingeschlossene Winkel (A) ungefähr 60 Grad beträgt und wobei der an der Hinterkante eingeschlossene Winkel (B) ungefähr 55 Grad beträgt.

2. Kolben nach einem der vorstehenden Ansprüche, wobei der Kolben (1) weiter eine hintere Dichtung (9) umfasst zum Abdichten eines Zwischenraums zwischen der Seitenwand (3) des Kolbenkörpers (2) und einer Wand der Kammer (21), wobei die hintere Dichtung (9) einen an der Vorderkante eingeschlossenen Winkel und einen an der Hinterkante eingeschlossenen Winkel hat, und wobei der an der Hinterkante eingeschlossene Winkel zwischen etwa 70 Grad und 90 Grad beträgt.

3. Kolben nach Anspruch 2, wobei der an der Hinterkante eingeschlossene Winkel der hinteren Dichtung (9) ungefähr 90 Grad beträgt.

4. Kolben nach einem der vorstehenden Ansprüche, wobei der Kolben (1) aus Polyethylen niedriger Dichte (LDPE) hergestellt ist und eine Shore-D-Härte zwischen 85 und 95 hat.

5. Kolben nach einem der vorstehenden Ansprüche, wobei die vordere Dichtung (5) um zwischen 0,8 mm und 1,4 mm aus der Seitenwand (3) des Kolbenkörpers (2) vorkragt.

6. Kolben nach einem der vorstehenden Ansprüche, wobei der Kolben (1') einen koaxialen Stift (14) umfasst, der aus der Frontseite des Kolbens herausragt.

7. Kolben nach einem der vorstehenden Ansprüche in Kombination mit einer Abgabekartusche (20), die die Kammer (21) einschließt.

8. Kombination nach Anspruch 7, wobei die Kammer (21) ein Material mit einer Viskosität zwischen 30 und 1500 Pa.s einschließt.

9. Kombination nach Anspruch 8, wobei das Material ein Dentalmaterial ist.

10. Vorrichtung zum Herauspressen eines Materials, umfassend:
(a) eine Kartusche (20) mit einer Kammer (21) zur Aufnahme des Materials und einer Öffnung (22), durch die das Material herausgepresst werden kann; und
(b) einen Kolben (1) nach einem der Ansprüche 1 bis 6, der zum Einsetzen in die und zur Hin- und Herbewegung in der Kammer (21) eingerichtet ist.

11. Vorrichtung nach Anspruch 10, weiter umfassend einen Stößel (14), der zum Vorrücken des Kolbens (1) eingerichtet ist, und wobei der Stößel (140) ein Zahnsegment umfasst, und wobei die Vorrichtung ein Aktorsystem zum Vorrücken des Stößels (140) umfasst, und wobei das Aktorsystem einen Vorrückmechanismus und einen Anschlag umfasst, und wobei das Aktorsystem weiter einen Hebel (110) zum manuellen Betätigen des Vorrückmechanismus umfasst, und wobei der Vorrückmechanismus dazu eingerichtet ist, eine Sperrklinke (111) abwechselnd in Vorhüben und Rückhüben zu bewegen, um den Stößel (140) während der Vorhübe schrittweise vorwärts zu bewegen.

12. Vorrichtung nach Anspruch 11, wobei der Vorrückmechanismus bewirkt, dass
(i) die Sperrklinke (111) während der Vorhübe in Eingriff mit dem Zahnsegment des Stößels (140) ist, und dass
(ii) die Sperrklinke (111) während der Rückhübe außer Eingriff mit dem Zahnsegment des Stößels (140) ist.

13. Vorrichtung nach Anspruch 11 und 12, wobei das Aktorsystem bewirkt, dass
(i) der Anschlag am Ende eines Rückhubs mit dem Zahnsegment des Stößels in Eingriff ist, und dass
(ii) der Anschlag während des größten Teils der Vor- und Rückhübe außer Eingriff mit dem Zahnsegment ist.

## Revendications

1. Piston (1) destiné à être utilisé pour expulser un matériau d'une chambre (21), comprenant :
(a) un corps de piston (2) comportant une paroi latérale (3) ; et
(b) un joint avant (5) destiné à sceller un écartement entre la paroi latérale (3) du corps de piston (2) et une paroi de la chambre (21), dans lequel le joint avant (5) comprend une crête annulaire (8) possédant profil généralement en forme de « V » s'effilant vers la périphérie externe du joint (5), et a un angle inclus de bord d'attaque (A), et un angle inclus de bord de fuite (B) qui est plus petit que l'angle inclus de bord d'attaque (A), les angles inclus de bords d'attaque et de fuite (A, B) étant mesurés par rapport à la paroi latérale (3) du piston, dans lequel le bord d'attaque (6) correspond au flanc de la partie en forme de « V » du profil de joint avant qui est conçu pour faire face au matériau dans la chambre (21), et le bord de fuite (7) est le bord opposé au bord d'attaque (6) conçu pour être tourné à l'écart du matériau dans la chambre (21), et dans lequel l'angle inclus de bord d'attaque (A) vaut approximativement 60 degrés, et dans lequel l'angle inclus de bord de fuite (B) vaut approximativement 55 degrés.

2. Piston selon l'une quelconque des revendications précédentes, où le piston (1) comprend en outre un joint arrière (9) destiné à sceller un écartement entre la paroi latérale (3) du corps de piston (2) et une paroi de la chambre (21), dans lequel le joint arrière (9) a un angle inclus de bord d'attaque et un angle inclus de bord de fuite, et l'angle inclus de bord de fuite est compris entre environ 70 degrés et 90 degrés.

3. Piston selon la revendication 2, dans lequel l'angle inclus de bord de fuite du joint arrière (9) est approximativement de 90 degrés.

4. Piston selon l'une quelconque des revendications précédentes, où le piston (1) est constitué de polyéthylène basse densité (LDPE) et a une dureté Shore D comprise entre 85 et 95.

5. Piston selon l'une quelconque des revendications précédentes, dans lequel le joint avant (5) fait saillie de la paroi latérale (3) du corps de piston (2) d'une valeur comprise entre 0,8 mm et 1,4 mm.

6. Piston selon l'une quelconque des revendications précédentes, où le piston (1') comprend une broche coaxiale (14) dépassant de l'avant du piston.

7. Piston selon l'une quelconque des revendications précédentes, en combinaison avec une cartouche de distributeur (20) qui inclut la chambre (21).

8. Combinaison selon la revendication 7, dans laquelle la chambre (21) inclut un matériau avec une viscosité comprise entre 30 et 1500 Pa.s.

9. Combinaison selon la revendication 8, dans laquelle le matériau est un matériau dentaire.

10. Dispositif pour expulser un matériau, comprenant :
(a) une cartouche (20) comportant une chambre (21) pour contenir le matériau, et une ouverture (22) à travers laquelle le matériau peut être expulsé ; et
(b) un piston (1) selon l'une quelconque des revendications 1 à 6, conçu pour une insertion et un mouvement de va-et-vient à l'intérieur de la chambre (21).

11. Dispositif selon la revendication 10, comprenant en outre un mécanisme coulissant (14) conçu pour faire avancer le piston (1), et dans lequel le mécanisme coulissant (140) comprend un rochet, et le dispositif comprend un système d'actuateur permettant de faire avancer le mécanisme coulissant (140), et dans lequel le système d'actuateur comprend un mécanisme d'avancement et une butée, et dans lequel le système d'actuateur comprend en outre un levier (110) pour l'actionnement manuel du mécanisme d'avancement, et dans lequel le mécanisme d'avancement est conçu pour déplacer en alternance un cliquet (111) dans des courses vers l'avant et de retour pour un déplacement par étapes du mécanisme coulissant (140) vers l'avant pendant les courses vers l'avant.

12. Dispositif selon la revendication 11, dans lequel le mécanisme d'avancement assure que
(i) le cliquet (111) est en prise avec le rochet du mécanisme coulissant (140) pendant les courses vers l'avant, et que
(ii) le cliquet (111) est désolidarisé du rochet du mécanisme coulissant (140) pendant les courses de retour.

13. Dispositif selon les revendications 11 et 12, dans lequel le système d'actuateur assure que
(i) la butée est en prise avec le rochet du mécanisme coulissant à la fin d'une course de retour, et que
(ii) la butée est désolidarisée du rochet pendant la majeure partie des courses vers l'avant et de retour.
